# EUROPÄISCHE PATENTSCHRIFT

(11) **EP 4 014 918 B1**
(45) Veröffentlichungstag und Bekanntmachung des Hinweises auf die Patenterteilung: **26.04.2023**
(21) Anmeldenummer: 20215895.2
(22) Anmeldetag: 21.12.2020
(51) Int. Cl.: A61C 1/00

(54) **SYSTEM ZUM EXKAVIEREN VON ZAHNMATERIAL**
SYSTEM FOR EXCAVATING DENTAL MATERIAL
SYSTÈME D'EXCAVATION DE MATIÈRE DENTAIRE

(43) Veröffentlichungstag der Anmeldung: 22.06.2022
(73) Patentinhaber: Ivoclar Vivadent AG, 9494 Schaan (LI)
(72) Erfinder: HAUNER-WESTPHAL, Martina, 6800 Feldkirch (AT); PESCHKE, Arnd, 9498 Planken (LI); BOCK, Thorsten, 6800 Feldkirch (AT)
(74) Vertreter: Baldus, Oliver

(56) Entgegenhaltungen:
- WO-A1-2011/107117
- DE-A1- 4 343 218
- US-A1- 2015 044 632

## Beschreibung

Die vorliegende Erfindung betrifft ein System zum Exkavieren von Zahnmaterial nach Anspruch 1.

Aus DE 43 43 218 A1 ist eine Vorrichtung bekannt zum Entfernen von Ablagerungen auf einem Zahn mit einer Laserlichtquelle und mit einem Laserlichtstrahl, der in einen Flüssigkeitsstrahl eingekoppelt ist und koaxial mit dem Flüssigkeitsstrahl austritt, wobei der von dem laserlichtleitenden Flüssigkeitsstrahl entgegen der Durchflußrichtung der Flüssigkeit übertragene Körperschall das akustische Signal für einen akustischen Sensor überträgt.

Aus WO 2011/107117 A1 ist ein Lasersystem zur ablativen Behandlung von Körpergewebe bekannt, das eine Laserquelle zur Erzeugung eines Laserstrahls, einen Scanner für den Laserstrahl, eine Steuereinheit für die Laserquelle und den Scanner, ein Handstück und mindestens eine am Handstück angebrachte Schallempfangsvorrichtung zum Empfang von Schall, der vom Laserstrahl beim Auftreffen auf das Körpergewebe erzeugt wird, umfasst.

Ein System zur Behandlung eines Zahns mit einem kariösen Bereich mit einem Flüssigkeitsstrahl ist aus US 2015/044632 A1 bekannt.

Bei der Exkavation, d.h. bei der Entfernung, von kariösem Zahnmaterial aus einem Zahn mittels eines Hochdruck-Fluidstrahls kann es vorkommen, dass unbeabsichtigt zu viel Zahnmaterial abgetragen wird. Hierbei kann es sogar vorkommen, dass es zu einer Pulpenperforation kommt, bei dem der Nerv des Zahns irreversibel geschädigt wird.

Es ist daher die technische Aufgabe der vorliegenden Erfindung, eine ungewünschte Abtragung von Zahnmaterial während eines Exkavationsprozesses mittels eines Fluidstrahls zu verhindern.

Diese Aufgabe wird durch den Gegenstand des unabhängigen Anspruchs 1 gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren (nicht beansprucht) zum Exkavieren von Zahnmaterial gelöst, mit den Schritten eines Anordnens eines Körperschallsensors auf einem Zahn; eines Exkavierens des Zahnmaterials mittels eines Hochdruck-Fluidstrahls; eines Erfassens von Körperschallsignalen während des Exkavierens durch den Körperschallsensor; und eines Regelns oder Steuerns einer Fluidstrahlerzeugungsvorrichtung auf Basis der Körperschallsignale. Durch die Rückkopplung über die Körperschallsignale sind Unterschiede im Hartgewebe des Zahns detektierbar. Auf diese Weise kann beispielsweise die Qualität des Fluidstrahls an denjenigen Bereich angepasst werden, der gerade vom Fluidstrahl bearbeitet wird. Zudem kann der Fluidstrahl automatisch deaktiviert werden, wenn dieser auf ein bestimmtes Material trifft. Eine Pulpenperforation kann auf diese Weise verhindert werden.

In einer technisch vorteilhaften Ausführungsform des Verfahrens wird der Fluidstrahl in Abhängigkeit der Körperschallsignale deaktiviert. Dadurch wird beispielsweise der technische Vorteil erreicht, dass eine ungewünschte und übermäßige Entfernung von Zahnmaterial verhindert wird.

In einer weiteren technisch vorteilhaften Ausführungsform des Verfahrens wird ein kontinuierlicher oder ein gepulster Fluidstrahl in Abhängigkeit der Körperschallsignale erzeugt. Dadurch wird beispielsweise der technische Vorteil erreicht, dass die Prozesseigenschaften des Fluidstrahls auf die Art des Zahnmaterials eingestellt werden können.

In einer weiteren technisch vorteilhaften Ausführungsform des Verfahrens wird ein Druck der Fluidstrahlerzeugung in Abhängigkeit der Körperschallsignale geregelt oder gesteuert. Dadurch wird beispielsweise der technische Vorteil erreicht, dass der Druck des Fluidstrahls auf die Art des Zahnmaterials eingestellt werden kann.

In einer weiteren technisch vorteilhaften Ausführungsform des Verfahrens wird die Art des Zahnmaterials auf Basis einer Steigung einer kumulierten Energie von Bursts erkannt. Dadurch wird beispielsweise der technische Vorteil erreicht, dass die Art des Zahnmaterial zuverlässig anhand der Steigungswerte erkannt werden kann.

In einer weiteren technisch vorteilhaften Ausführungsform des Verfahrens wird die Art des Zahnmaterials auf Basis einer Anzahl von Bursts je Zeiteinheit erkannt. Dadurch wird beispielsweise ebenfalls der technische Vorteil erreicht, dass die Art des Zahnmaterials schnell und zuverlässig erkannt werden kann.

In einer weiteren technisch vorteilhaften Ausführungsform des Verfahrens wird kariöses Zahnmaterial auf Basis der Frequenzen der Bursts erkannt. Dadurch wird beispielsweise ebenfalls der technische Vorteil erreicht, dass abzutragendes Zahnmaterial mit hoher Genauigkeit identifiziert werden kann.

In einer weiteren technisch vorteilhaften Ausführungsform des Verfahrens wird der Fluidstrahl deaktiviert, wenn kein kariöses Zahnmaterial mehr erkannt wird. Dadurch wird beispielsweise ebenfalls der technische Vorteil erreicht, dass lediglich kariöses Zahnmaterial abgetragen wird.

In einer weiteren technisch vorteilhaften Ausführungsform des Verfahrens werden die Körperschallsignale mit einer Sampling-Rate von bis zu 1 MHz erfasst. Dadurch wird beispielsweise ebenfalls der technische Vorteil erreicht, dass eine breite Datengrundlage für die Erfassung von Körperschallsignalen zur Steuerung des Fluidstrahls gewonnen werden kann.

Gemäß einem zweiten Aspekt wird die technische Aufgabe durch ein System zum Exkavieren von Zahnmaterial gelöst, mit einem Körperschallsensor zum Erfassen von Körperschallsignalen während des Exkavierens; einer Fluidstrahlerzeugungsvorrichtung zum Erzeugen eines Fluidstrahls zum Exkavieren des Zahnmaterials; und einer Vorrichtung zum Regeln oder Steuern der Fluidstrahlerzeugungsvorrichtung auf Basis der Körperschallsignale. Eine Auswerteeinheit ist in der Lage, in den Körperschallsignalen einzelne Bursts zu bestimmen und auszuwerten. Die Auswerteeinheit ist in der Lage, die Frequenz und die Energie der Bursts zu bestimmen. Durch das System werden die gleichen technischen Vorteile wie durch das Verfahren nach dem ersten Aspekt erreicht.

In einer weiteren technisch vorteilhaften Ausführungsform des Systems ist die Vorrichtung ausgebildet, die Fluidstrahlerzeugungsvorrichtung derart zu regeln oder zu steuern, dass der Fluidstrahl in Abhängigkeit der Körperschallsignale deaktiviert wird, ein kontinuierlicher oder ein gepulster Fluidstrahl in Abhängigkeit der Körperschallsignale erzeugt wird und/oder der Druck der Fluidstrahlerzeugung in Abhängigkeit der Körperschallsignale geregelt oder gesteuert wird. Dadurch wird beispielsweise ebenfalls der technische Vorteil erreicht, dass die Prozesseigenschaften des Fluidstrahls auf die Art des Zahnmaterials eingestellt werden können.

In einer weiteren technisch vorteilhaften Ausführungsform des Systems ist die Vorrichtung ausgebildet, die Art des Zahnmaterials auf Basis einer Steigung einer kumulierten Energie von Bursts zu erkennen. Dadurch wird beispielsweise ebenfalls der technische Vorteil erreicht, dass die Art des Zahnmaterials zuverlässig erkannt werden kann.

In einer weiteren technisch vorteilhaften Ausführungsform des Systems ist die Vorrichtung ausgebildet, die Art des Zahnmaterials auf Basis einer Anzahl von Bursts je Zeiteinheit zu erkennen. Dadurch wird beispielsweise ebenfalls der technische Vorteil erreicht, dass die Art des Zahnmaterials zuverlässig erkannt werden kann.

In einer weiteren technisch vorteilhaften Ausführungsform des Systems ist die Vorrichtung ausgebildet, kariöses Zahnmaterial auf Basis einer Frequenz von Bursts zu erkennen. Dadurch wird beispielsweise der technische Vorteil erreicht, dass der Fluidstrahl lediglich zur Entfernung von kariösem Zahnmaterial verwendet werden kann.

In einer technisch vorteilhaften Ausführungsform des Systems weist eine Auswerteeinheit des Körperschallsensors eine Sampling-Rate von bis zu 1 MHz auf. Dadurch wird beispielsweise ebenfalls der technische Vorteil erreicht, dass eine breite Datengrundlage für die Erfassung von Körperschallsignalen zur Regelung oder Steuerung des Fluidstrahls gewonnen werden kann.

Ausführungsbeispiele der Erfindung sind in den Zeichnungen dargestellt und werden im Folgenden näher beschrieben.

Es zeigen:
- Fig. 1: eine schematische Ansicht eines Systems zum Exkavieren von Zahnmaterial;
- Fig. 2: ein beispielhaftes Diagramm von Körperschallsignalen;
- Fig. 3: ein Diagramm zur Auswertung der Körperschallsignale;
- Fig. 4: ein weiteres Diagramm zur Auswertung der Körperschallsignale;
- Fig. 5: ein weiteres Diagramm zur Auswertung der Körperschallsignale;
- Fig. 6: ein weiteres Diagramm zur Auswertung der Körperschallsignale;
- Fig. 7: ein weiteres Diagramm zur Auswertung der Körperschallsignale von kariösem Zahnmetrial; und
- Fig. 8: ein Blockdiagramm eines Verfahrens (nicht beansprucht) zum Exkavieren von Zahnmaterial.

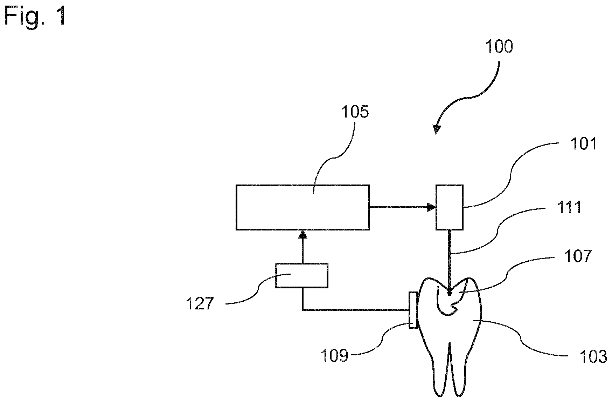

Fig. 1 zeigt eine schematische Ansicht eines Systems 100 zum Exkavieren von Zahnmaterial 107. Das System 100 umfasst eine Fluidstrahlerzeugungsvorrichtung 101 zum Erzeugen eines Fluidstrahls 111 zum Exkavieren von Zahnmaterial 107 des Zahnes 103. Der Fluidstrahl ist beispielsweise ein Wasserstrahl oder ein Strahl aus Natriumclorid- oder auch Ringerlösung. Ein Körperschallsensor 109 dient zum Erfassen von Körperschallsignalen während dem Prozess des Exkavierens. Der Körperschallsensor 109 ist zu diesem Zweck mit dem Zahn 103 derart gekoppelt, dass dieser die Körperschallwellen des Zahns 103 erfassen kann, die durch den Fluidstrahl 111 erzeugt werden. Die so gesammelten Körperschallsignale werden mittels der Auswerteeinheit 127 verstärkt, ausgewertet und elektronisch an eine elektronische Vorrichtung 105 zur Regelung oder Steuerung mit einem Mikroprozessor übertragen.

Diese Vorrichtung 105 wertet die Informationen der Auswerteeinheit 127 aus und steuert auf Basis dieser gewonnenen Kenngrößen die Fluidstrahlerzeugungsvorrichtung 101. Hierdurch wird ein geschlossener Regelkreis für den Fluidstrahl 111 realisiert.

Durch die Auswertung der Körperschallsignale sind Unterschiede im Hartgewebe des Zahns 103 detektierbar. Beispielsweise kann auf Basis der Körperschallsignale ermittelt werden, ob mittels des Fluidstrahls 111 ein Schmelzbereich, ein Dentinbereich oder ein kariöser Bereich des Zahns 103 bearbeitet wird.

Der bei einem Druck von 1 bis 500 bar in einer Düse der Fluidstrahlerzeugungsvorrichtung 101 generierte Fluidstrahl 111 weist beispielsweise einen Strahldurchmesser von 0,08 bis 0,3 mm auf. Der Fluidstrahl 111 kann dabei als kontinuierlicher Strahl austreten oder als ein pulsierender Strahl. Der pulsierende Fluidstrahl 111 kann je nach Frequenz bei der Pulsanregung aus 20 bis 40000 getrennten Tropfen- oder Fluidpaketen pro Sekunde aufgebaut werden. Das entspricht einer Tropfenfrequenz von 20 Hz bis 40 kHz.

Die Fluidstrahlerzeugungsvorrichtung 101 kann elektronisch geregelt oder gesteuert werden, um beispielsweise den Druck der Fluidstrahlgenerierung 111 zu verändern oder den Fluidstrahl 111 gezielt ein oder auszuschalten. Zudem kann die Fluidstrahlerzeugungsvorrichtung 101 derart geregelt oder gesteuert werden, dass diese einen kontinuierlichen oder einen pulsierenden Fluidstrahl 111 erzeugt oder den Druck zur Generierung des Fluidstrahls verändert. Zu diesem Zweck ist die Fluidstrahlerzeugungsvorrichtung 101 mit der Vorrichtung 105 gekoppelt.

Der Körperschallsensor 109 ist ein aktiver oder passiver Sensor und wird an demjenigen Zahn befestigt, an dem die Exkavation des Zahnmaterials 107 vorgenommen wird. Der Körperschall kann auch über den Kiefer auf einen anderen Zahn oder den Kiefer selbst übertragen werden und ist dort gemessen werden. Der Körperschallsensor 109 ist in der Lage, die weitergeleiteten Schallsignale des Zahns 103 im Körperschallbereich während der Exkavation mittels des Fluidstrahls 111 zu erfassen. Die Auswerteeinheit 127 arbeitet beispielsweise mit einer Sampling-Rate von 1 MHz, so dass sich Körperschallsignale des Zahns 103 bis zu einer Frequenz von 500 kHz erfassen lassen. Im Allgemeinen kann der Körperschallsensor 109 mit zugehöriger Auswerteeinheit 127 jedoch auch ausgebildet sein, um Signale in anderen Frequenzbereichen zu messen.

Die elektrischen Körperschallsignale des Körperschallsensors 109 können durch einen elektronischen Verstärker verstärkt werden, so dass sich diese besser durch die Auswerteeinheit 127 auswerten und in der Vorrichtung 105 weiterverarbeiten lassen. Der Verstärker und/oder die Auswerteeinheit 127 kann zusätzlich einen Analog/Digitalwandler umfassen, so dass die gewonnenen Messwerte direkt an die digitale Vorrichtung 105 übermittelt werden können und von dieser verarbeitet werden können. Hierzu kann ein Computerprogramm von der Auswerteeinheit 127 und/oder Vorrichtung 105 ausgeführt werden oder die Regelungseinheit 105 umfasst eine geeignete Auswerteeinheit selbst.

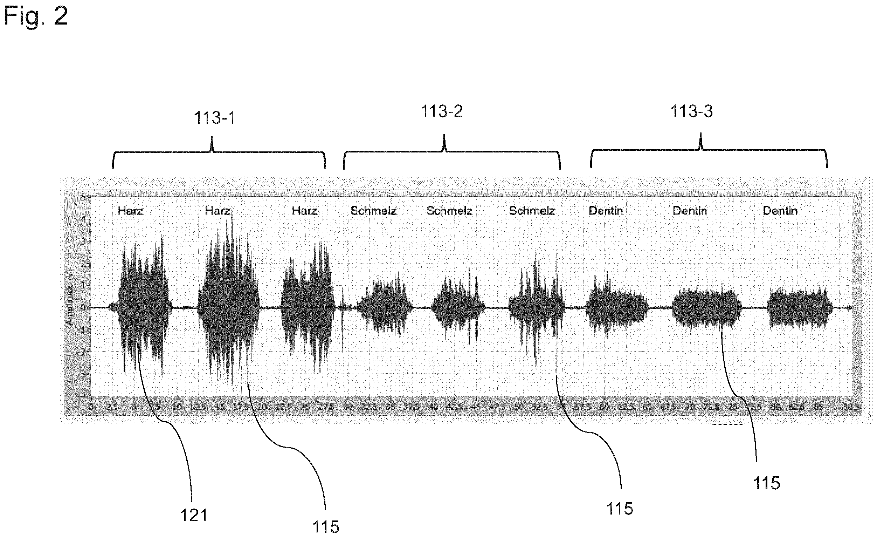

Fig. 2 zeigt ein beispielhaftes Diagramm von Körperschallsignalen 121, die von dem Körperschallsensor 109 bei der Bearbeitung einer Zahnprobe in unterschiedlichen Zahnbereichen erhalten werden. Das Diagramm zeigt die erfasste Amplitude der Körperschallsignale 121 in Abhängigkeit der Zeit oder der gewonnenen Samples in drei unterschiedlichen Zeitbereichen 113-1, ..., 113-3.

Im ersten Zeitbereich 113-1 trifft der bei einem Druck von 250 bar erzeugte Fluidstrahl 111 auf einen Kunstharzbereich der eingebetteten Zahnprobe. Im Zeitbereich 113-2 trifft der gleiche Fluidstrahl 111 auf einen Schmelzbereich des Zahnes 103 und im Zeitbereich 113-3 trifft der gleiche Fluidstrahl 111 auf einen Dentinbereich des Zahnes 103.

Die Körperschallsignale 121 unterscheiden sich, je nachdem auf welches Material der Fluidstrahl 111 auftrifft. Daher treten in jedem Zeitbereich 113-1, ..., 113-3 unterschiedliche Amplitudenspitzen (s.g. Bursts) 115 auf, aus denen Rückschlüsse auf die Art und den Härtegrad des bearbeiteten Zahnmaterials 107 gewonnen werden können. Ein Burst 115 entsteht bei der Abtragung von Zahnmaterial 107 durch den Fluidstrahl 111 und verursacht ein Klirren oder Knacken im Körperschallbereich. Ein Burst 115 stellt sich im Signal-ZeitDiagramm als Peak (Spitze) dar. Mittels einer Körperschallmessung und der digitalen Auswertung der entstehenden kurzzeitigen Bursts 115 kann zwischen den unterschiedlichen Zahnhartsubstanzen unterschieden werden, wie beispielsweise unterschiedlichen Zahnmaterialien oder Füllungsmaterialien. Dies kann wiederum für eine Regelung oder Steuerung des Fluidstrahls 111 verwendet werden.

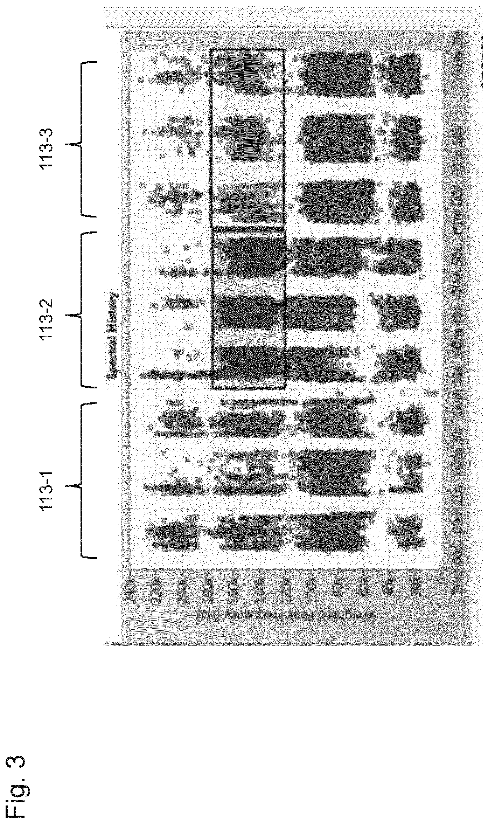

Fig. 3 zeigt ein Diagramm zur Auswertung der Körperschallsignale 121. In diesem Diagramm ist die gewichtete Frequenz aller Bursts 115 gezeigt, die bei der Bearbeitung der Probe mittels des Fluidstrahls 111 in den unterschiedlichen Zeitbereichen 113-1, ..., 113-3 über die Zeit erhalten werden. Die gewichtete Frequenz der auftretenden Bursts 115 lässt sich beispielsweise mittels einer Fourier-Analyse aus dem zeitabhängigen Körperschallsignal 121 erhalten. Die Gewichtung kann anhand des Schwerpunkts des Frequenzspektrums erfolgen.

In jedem Zeitbereich 113-1, ..., 113-3 tritt eine andere Verteilung der gewichteten Frequenzen der Bursts 115 auf, je nachdem welche Art von Zahnmaterial 107 gerade von dem Fluidstrahl 111 getroffen wird.

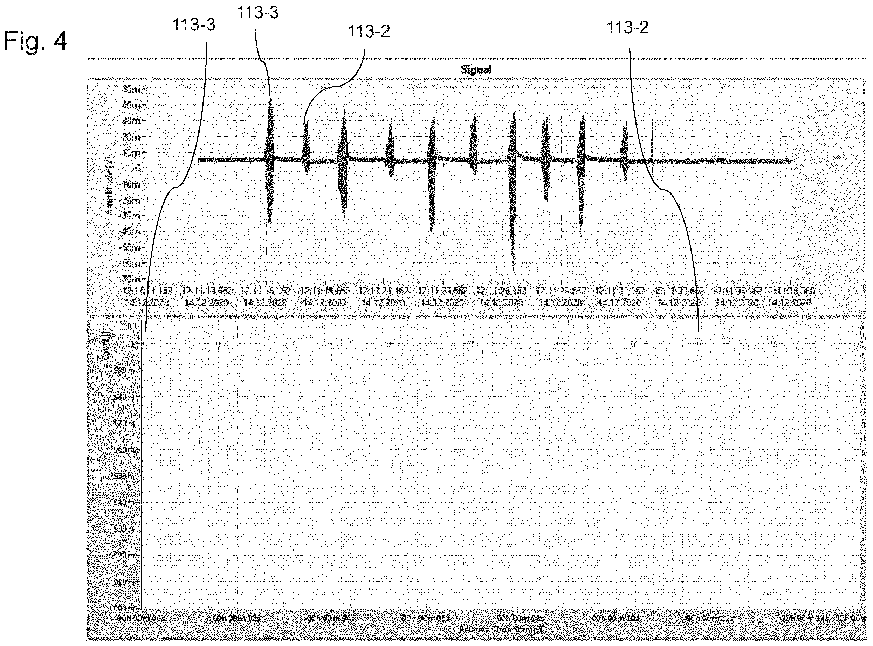

Fig. 4 zeigt ein weiteres Diagramm zur Auswertung und Clusterung der Körperschallsignale, bei dem Dentin 113-3 und Zahnschmelz 113-2 bearbeitet werden. Im Dentin ist die Signalamplitude höher als im Schmelzbereich des Zahns. Nach Clusterung der Bursts z.B. auf Basis der gewählten Parameter Partial Power, Weighted Peak Frequency, Frequency Centroid Energy und Peak Signal kann eindeutig zwischen Dentin 113-3 (Kreise) und Zahnschmelz 113-2 (Kreise) unterschieden werden.

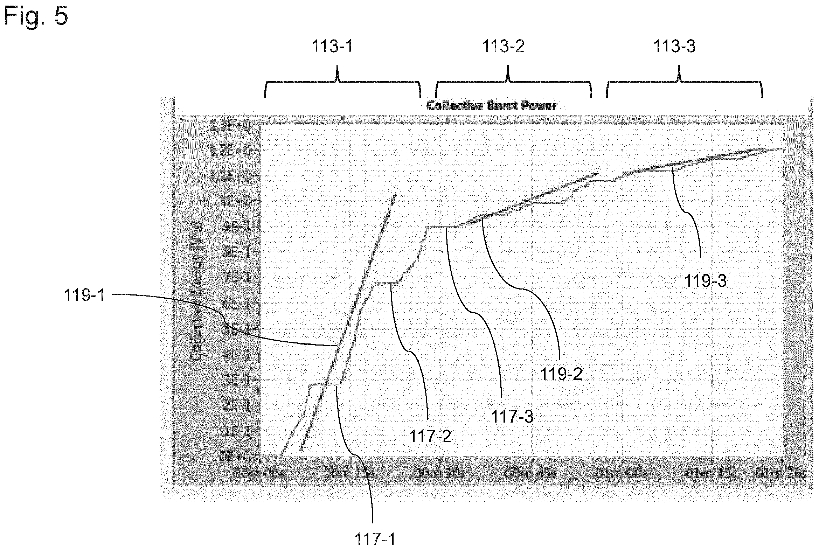

Fig. 5 zeigt ein weiteres Diagramm zur Auswertung der Körperschallsignale 121. In diesem Diagramm ist die kumulierte Energie der Bursts 115 im Laufe der Zeit gezeigt. In dem Bereich 113-1, in dem der Fluidstrahl 111 auf den Kunstharzbereich der eingebetteten Zahnprobe trifft weist die kumulierte Energie der Bursts 115 eine andere Steigung je Zeiteinheit auf als in dem Bereich 113-2, in dem der Fluidstrahl 111 auf einen Schmelzbereich des Zahnes 103 trifft oder in dem Bereich 113-3, in dem der Fluidstrahl 111 auf einen Dentinbereich des Zahnes 103 trifft. Je nach der Steigung im Diagramm 119-1, ..., 119-3 der kumulierten Energie der Bursts 115 kann daher bestimmt werden, welche Art von Zahnmaterial des Zahnes 103 gerade von dem Fluidstrahl 111 bearbeitet wird. In den horizontalen Bereichen 117-1, ..., 117-3 findet keine Bearbeitung durch den Fluidstrahl 111 statt. Die kumulierte Energie der Bursts 115 steigt in diesen Zeitbereichen daher nicht.

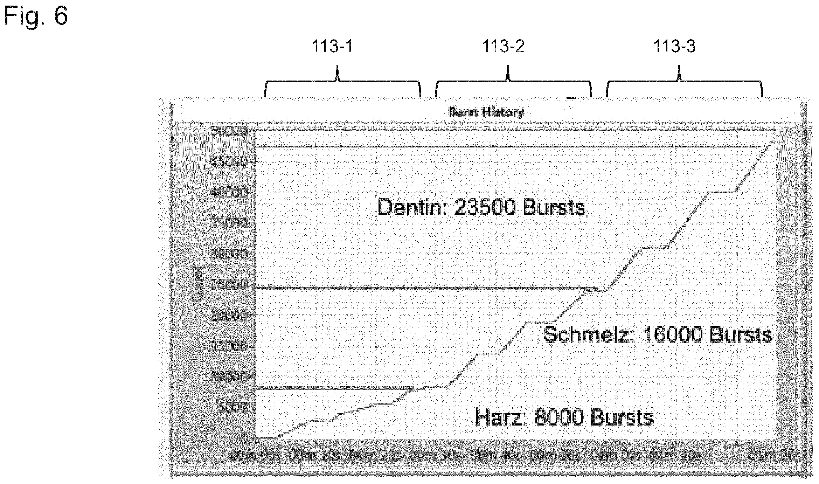

Fig. 6 zeigt ein weiteres Diagramm zur Auswertung der Körperschallsignale 121. In diesem Diagramm ist die kumulierte Anzahl der Bursts 115 in Abhängigkeit der Zeit aufgetragen. Bei der Bearbeitung des Kunstharzes im Zeitbereich 113-1 werden 8.000 Bursts gezählt. Bei der Bearbeitung des Zahnschmelzbereiches im Zeitbereich 113-2 werden 16.000 Bursts gezählt und bei der Bearbeitung im Zeitbereich 113-3 werden 23.500 Bursts gezählt. Anhand der Anzahl der Bursts 115 je Zeiteinheit kann daher festgestellt werden, um welche Art des Zahnmaterials es sich handelt. Auf Basis der Anzahl der Bursts je Zeiteinheit kann bestimmt werden, auf welche Art von Zahnmaterial 107 der Fluidstrahl 111 auftrifft.

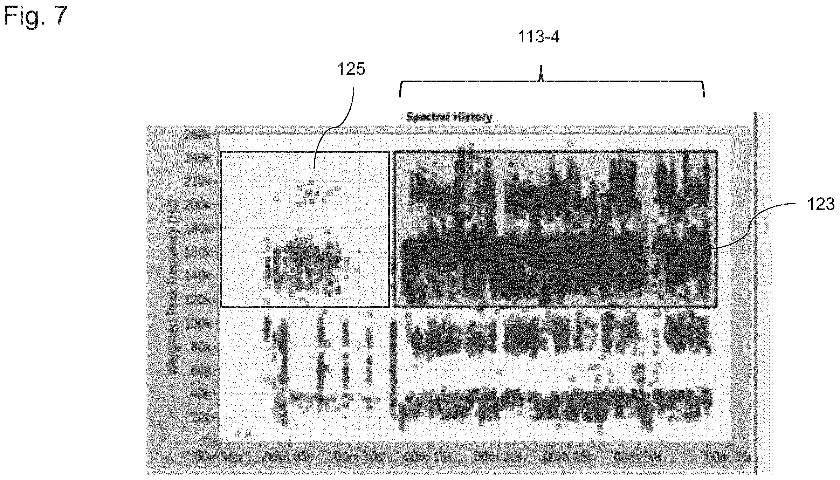

Fig. 7 zeigt ein weiteres Diagramm zur Auswertung der Körperschallsignale 121 von kariösem Zahnmaterial 107. In diesem Diagramm ist ebenfalls die gewichtete Frequenz aller Bursts 115 gezeigt, die bei der Bearbeitung des Zahns 103 mittels des Fluidstrahls 111 über die Zeit erhalten werden. Trifft der Fluidstrahl 111 auf ein kariöses Zahnmaterial 107 im Bereich 113-4 so treten mehr Bursts mit höheren gewichteten Frequenzen im Bereich 123 auf. Im Gegensatz dazu treten im Bereich 125 für gesundes Zahnmaterial weniger Bursts mit höheren gewichteten Frequenzen auf.

Durch die Auswertung der Körperschallsignale 121 kann somit auch die Bearbeitung von kariösem Zahnmaterial 107 erfasst werden. In diesem Fall tritt eine erhöhte Burstbildung im oberen Frequenzbereich auf. Dies kann dazu genutzt werden, die Bearbeitung mit dem Fluidstrahl 111 nur solange durchzuführen, bis das kariöse Zahnmaterial 107 entfernt worden ist. Sobald der Fluidstrahl 111 auf gesundes Zahnmaterial 107 trifft verschieben sich die Frequenzen der Bursts 115 in den unteren Frequenzbereich. In diesem Fall kann der Fluidstrahl 111 automatisch deaktiviert werden. Dadurch wird die Entfernung von gesundem Zahnmaterial 107 weitestgehend verhindert.

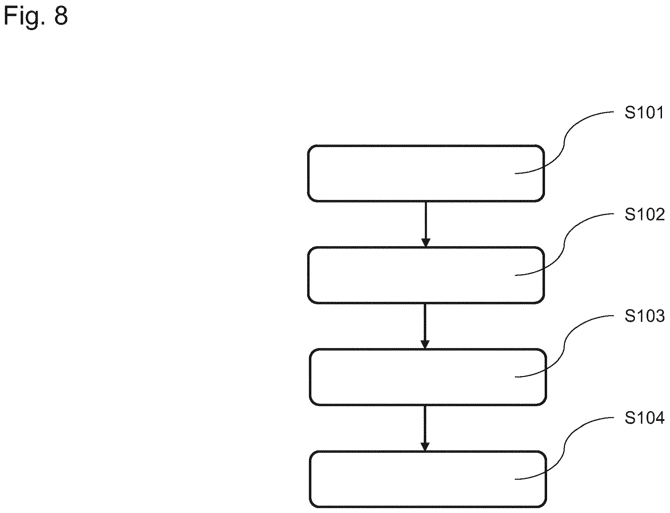

Fig. 8 zeigt ein Blockdiagram eines Verfahrens zum Exkavieren von Zahnmaterial 107. Zunächst wird in Schritt S101 ein Körperschallsensor 109 auf dem Zahn 103 befestigt. Im Schritt S102 wird das Zahnmaterial 107 mittels des Fluidstrahls 111 entfernt (exkaviert). Zeitgleich werden in Schritt S103 die Körperschallsignale 121 während des Exkavierens durch den Körperschallsensor 109 erfasst. Die Körperschallsignale 121 können zusätzlich verstärkt und ausgewertet werden. Danach wird im Schritt S104 die Fluidstrahlerzeugungsvorrichtung 101 auf Basis der Körperschallsignale 121 geregelt oder gesteuert.

Zur Regelung oder Steuerung der Fluidstrahlerzeugungsvorrichtung 101 wird neben dem Zeitsignal der Körperschallsignale 121 die zeitlich veränderliche Burstentwicklung (Counts) sowie deren zeitliche Energieentwicklung (-summierung) und deren Frequenzlage berücksichtigt. Die Auswerteeinheit 127 ist in der Lage, autonom die Frequenz, die Energie und/oder die Anzahl der Bursts 115 zu bestimmen.

Durch dieses Verfahren lässt sich erkennen, welches Material gerade von dem Fluidstrahl 111 bearbeitet wird. Je nach Material kann dann durch die Fluidstrahlerzeugungsvorrichtung 101 der Fluidstrahl 111 in seinen Eigenschaften verändert oder vollständig deaktiviert werden. Auf diese Weise lässt sich eine unbeabsichtigte Exkavation von Zahnmaterial 107 oder ein Pulpadurchschuss vermeiden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmalen nicht beschränkt.

### BEZUGSZEICHENLISTE

- 100: System zum Exkavieren von Zahnmaterial
- 101: Fluidstrahlerzeugungsvorrichtung
- 103: Zahn
- 105: Vorrichtung zum Regeln oder Steuern
- 107: Zahnmaterial
- 109: Körperschallsensor
- 111: Fluidstrahl
- 113: Zeitbereich
- 115: Bursts
- 117: Bereich
- 119: Bereich
- 121: Körperschallsignale
- 123: Bereich kariöses Zahnmaterial
- 125: Bereich gesundes Zahnmaterial
- 127: Auswerteeinheit

## Patentansprüche

1. System (100) zum Exkavieren von Zahnmaterial (107), mit:
- einem Körperschallsensor (109) zum Erfassen von Körperschallsignalen (121) während des Exkavierens, **gekennzeichnet durch**
- eine Fluidstrahlerzeugungsvorrichtung (101) zum Erzeugen eines Fluidstrahls (111) zum Exkavieren des Zahnmaterials (107); und
- eine Vorrichtung (105) zum Regeln oder Steuern der Fluidstrahlerzeugungsvorrichtung (101) auf Basis der Körperschallsignale (121).

2. System (100) nach Anspruch 1, wobei die Vorrichtung (105) ausgebildet ist, die Fluidstrahlerzeugungsvorrichtung (101) derart zu regeln oder steuern, dass der Fluidstrahl (111) in Abhängigkeit der Körperschallsignale (121) deaktiviert wird, ein kontinuierlicher oder ein gepulster Fluidstrahl (111) in Abhängigkeit der Körperschallsignale (121) erzeugt wird und/oder ein Druck der Fluidstrahlerzeugung in Abhängigkeit der Körperschallsignale (121) geregelt oder gesteuert wird.

3. System (100) nach Anspruch 1 oder 2, wobei die Vorrichtung (105) ausgebildet ist, die Art des Zahnmaterials (107) auf Basis einer Steigung einer kumulierten Energie von Bursts (115) zu erkennen.

4. System (100) nach einem der vorangehenden Ansprüche, wobei die Vorrichtung (105) ausgebildet ist, die Art des Zahnmaterials (107) auf Basis einer Anzahl von Bursts (115) je Zeiteinheit zu erkennen.

5. System (100) nach einem der vorangehenden Ansprüche, wobei die Vorrichtung (105) ausgebildet ist, kariöses Zahnmaterial (107) auf Basis einer Frequenz von Bursts (115) zu erkennen.

6. System (100) nach einem der vorangehenden Ansprüche, wobei eine Auswerteeinheit (127) des Systems (100) eine Sampling-Rate von bis zu 1 MHz aufweist.

## Claims

1. A system (100) for excavating dental material (107), having:
- a structure-borne sound sensor (109) for detecting structure-borne sound signals (121) during excavation, **characterized by**
- a fluid jet generating device (101) for generating a fluid jet (111) for excavating the tooth material (107); and
- a device (105) for regulating or controlling the fluid jet generating device (101) based on the structure-borne sound signals (121).

2. The system (100) according to claim 1, wherein the device (105) is configured to regulate or control the fluid jet generating device (101) such that the fluid jet (111) is deactivated in dependence on the structure-borne sound signals (121), a continuous or a pulsed fluid jet (111) is generated in dependence on the structure-borne sound signals (121), and/or a pressure of the fluid jet generation is regulated or controlled in dependence on the structure-borne sound signals (121).

3. The system (100) according to claim 1 or 2, wherein the device (105) is configured to detect the type of tooth material (107) based on a slope of a cumulative energy of bursts (115).

4. The system (100) according to any one of the preceding claims, wherein the device (105) is configured to detect the type of tooth material (107) based on a number of bursts (115) per unit of time.

5. The system (100) according to any one of the preceding claims, wherein the device (105) is configured to detect carious tooth material (107) based on a frequency of bursts (115).

6. The system (100) according to any one of the preceding claims, wherein an evaluation unit (127) of the system (100) has a sampling rate of up to 1 MHz.

## Revendications

1. Système (100) pour excaver du matériau dentaire (107), comprenant :
- un capteur de bruit de structure (109) pour détecter des signaux de bruit de structure (121) pendant l'excavation, **caractérisé par**
- un dispositif de génération de jet de fluide (101) pour générer un jet de fluide (111) pour excaver le matériau dentaire (107) ; et
- un dispositif (105) pour commander ou contrôler le dispositif de génération de jet de fluide (101) sur la base des signaux de bruit de structure (121).

2. Système (100) selon la revendication 1, où le dispositif (105) est conçu pour réguler ou commander le dispositif de génération de jet de fluide (101) de telle manière à désactiver le jet de fluide (111) en fonction des signaux acoustiques de structure (121), à générer un jet de fluide continu ou pulsé (111) en fonction des signaux acoustiques de structure (121) et/ou régler ou commander une pression de génération de jet de fluide en fonction des signaux acoustiques de structure (121).

3. Système (100) selon la revendication 1 ou 2, où le dispositif (105) est adapté pour reconnaître le type de matériau dentaire (107) sur la base d'une pente d'une énergie cumulée de rafales (115).

4. Système (100) selon l'une des revendications précédentes, où le dispositif (105) est adapté pour reconnaître le type de matériau dentaire (107) sur la base d'un nombre de rafales (115) par unité de temps.

5. Système (100) selon l'une des revendications précédentes, dans lequel le dispositif (105) est adapté pour détecter un matériau dentaire carié (107) sur la base d'une fréquence de rafales (115).

6. Système (100) selon l'une des revendications précédentes, où une unité d'évaluation (127) du système (100) présente un taux d'échantillonnage allant jusqu'à 1 MHz.
